# EUROPÄISCHE PATENTSCHRIFT

(11) **EP 0 975 783 B1**
(45) Veröffentlichungstag und Bekanntmachung des Hinweises auf die Patenterteilung: **31.10.2001**
(21) Anmeldenummer: 98916814.1
(22) Anmeldetag: 27.02.1998
(51) Int. Cl.: C12N 15/86, C12N 5/10, A61K 48/00

(54) **RETROVIRALE VEKTOREN, VERFAHREN ZU IHRER HERSTELLUNG UND IHRE VERWENDUNG ZUR GENÜBERTRAGUNG IN CD4-POSITIVE ZELLEN**
RETROVIRAL VECTORS, METHOD FOR THE PRODUCTION AND THE USE THEREOF FOR GENE TRANSFER IN CD4-POSITIVE CELLS
VECTEURS RETROVIRAUX, LEUR PROCEDE DE PRODUCTION ET LEUR UTILISATION POUR LE TRANSFERT GENIQUE DANS DES CELLULES POSITIVES CD-4

(30) Priorität: 27.02.1997 DE 19707971; 27.02.1998 DE 19808438
(43) Veröffentlichungstag der Anmeldung: 02.02.2000
(73) Patentinhaber: Bundesrepublik Deutschland, letztvertreten durch den Präsidenten des Paul-Ehrlich-Instituts, 63225 Langen (DE)
(72) Erfinder: CICHUTEK, Klaus, D-60598 Frankfurt am Main (DE); STITZ, Jörn, D-60318 Frankfurt am Main (DE)
(74) Vertreter: Vossius, Volker, Dr.
(86) Internationale Anmeldenummer: DE9800593
(87) Internationale Veröffentlichungsnummer: WO9838325

(56) Entgegenhaltungen:
- WO-A-96/30533
- SALMONS B. ET AL.: "CONSTRUCTION OF RETROVIRAL VECTORS FOR TARGETED DELIVERY AND EXPRESSION OF THERAPEUTIC GENES" LEUKEMIA, Bd. 9, Nr. SUPPL. 01, 1. Oktober 1995, Seiten S53-S60, XP000575934
- WILK T. ET AL.: "Retained in vitro activity and cytopathogenicity of HIV-1 despite truncation of the C-terminal tail of the env gene product." VIROLOGY, Bd. 189, 1992, Seiten 167-177, XP002074097 in der Anmeldung erwähnt
- COSSET F.-L. ET AL.: "HIGH-TITER PACKAGING CELLS PRODUCING RECOMBINANT RETROVIRUSES RESISTANT TO HUMAN SERUM" JOURNAL OF VIROLOGY, Bd. 69, Nr. 12, 1. Dezember 1995, Seiten 7430-7436, XP000569527 in der Anmeldung erwähnt
- MILLER N. ET AL.: "TARGETED VECTORS FOR GENE THERAPY" FASEB JOURNAL, Bd. 9, Nr. 2, Februar 1995, Seiten 190-199, XP000616414
- VON KALLE C. ET AL.: "Increased gene transfer into human hematopoietic progenitor cells by extended in vitro exposure to a pseudotyped retroviral vector." BLOOD, Bd. 84, Nr. 9, 1994, Seiten 2890-2897, XP000196799
- TAKEUCHI Y. ET AL.: "Retroviral pseudotypes produced by rescue of a Moloney murine leukemia virus vector by C-type, but not D-type, retroviruses." VIROLOGY, Bd. 186, 1992, Seiten 792-794, XP002074099
- SCHIERLE B. S. ET AL.: "Pseudotyping of murine leukemia virus with the envelope glycoproteins of HIV generates a retroviral vetor with specificity of infection for CD4-expressing cells." PROC. NATL. ACAD. SCI. USA, Bd. 94, August 1997, Seiten 8640-8645, XP002074100
- MAMMANO F. ET AL.: "Truncation of the human immunodeficiency virus type 1 envelope glycoprotein allows efficient pseudotyping of Moloney murine leukemia virus particles and gene transfer into CD4+ cells." JOURNAL OF VIROLOGY, Bd. 71, Nr. 4, April 1997, Seiten 3341-3345, XP002074198

## Beschreibung

Gegenstand der Erfindung sind retrovirale Vektoren (Zell-Targeting-Vektoren), Verfahren zu ihrer Herstellung und ihrer Verwendung zur Genübertragung in CD4-positive Zellen.

Der Ausdruck "retrovirale Vektoren" oder "retrovirale Transfervektoren" bezeichnet infektiöse aber vermehrungsunfähige Retroviren, die Gene in Form von retroviralen Expressionskonstrukten (auch Expressionsvektoren genannt) in Zellen einschleusen können. Die Genübertragung führt zur Integration des Expressionskonstrukts in das Genom der Zelle. Der retrovirale Gentransfer ist vorteilhaft, weil (i) in der Regel eine Kopie des gewünschten Gens in Zellen überführt wird, (ii) das Gen im allgemeinen ohne Mutationen oder Umlagerungen transferiert wird und (iii) ein stabiler chromosomaler Einbau erfolgt.

Es ist bekannt, retrovirale Vektoren auf der Basis des amphotropen murinen Leukämievirus (MLV) zu benutzen, um bestimmte Gene in Säugerzellen, insbesondere in humane Zellen, zu überführen. Diese Vektoren sind vermehrungsunfähig und durchlaufen lediglich eine Replikationsrunde. Zur Herstellung derartiger Vektoren benötigt man zwei Komponenten. Zum einen benötigt man eine Verpackungszelle, welche die *gag-*, *pol-* und *env*-Genprodukte des MLV durch Expression psi-negativer Konstrukte bereitstellt, so daß diese Gene nicht in ein Retrovirus verpackt werden können. Als "psi" wird das Verpackungssignal der Retroviren bezeichnet, das die effiziente Verpackung der Boten-RNA steuert. Zum anderen muß ein sogenanntes Expressionskonstrukt hergestellt werden, das eine Verpackung in den retroviralen Vektor und den Transfer durch das Retrovirus erlaubt und das eine kodierende und translatierbare Region des gewünschten Genprodukts enthält. Somit muß das Expressionskonstrukt das Verpackungssignal psi enthalten. Die in der unbehandelten Verpackungszelle befindlichen *gag-, pol-* und *env-*Gene müssen psi-negativ sein, damit entsprechende Boten-RNA nicht in die Retroviruspartikel aufgenommen wird. Nach der Überführung des Expressionskonstrukts durch Transfektion der entsprechenden Vektor-DNA in die Verpackungszellen werden in den Zellüberstand retrovirale Vektorpartikel abgegeben, die ausschließlich das Expressionskonstrukt enthalten, nicht jedoch die psi-negativen *gag*-, *pol-* und *env*-Gene, so daß diese Gene nicht in Zielzellen überführt werden.

Der Tropismus der retroviralen Vektoren, d. h. die Auswahl der Säugerzellen, in welche diese Vektoren das Expressionskonstrukt überführen können, wird durch das *env*-Gen in der benutzten Verpackungszelle bestimmt. Das *env*-Gen wird in Hüllproteine, das Transmembranprotein (TM) und das Oberflächenhüllprotein (SU) translatiert, welche die äußere Hülle des retroviralen Vektors bilden. Die *env*-Genprodukte des amphotropen MLV, das bisher vor allem für den Gentransfer benutzt wird, erlauben den Gentransfer in eine große Anzahl unterschiedlicher Säugerzellen. Speziell für den Gentransfer in humane Zellen erlaubt der amphotrope retrovirale Vektor jedoch nicht den selektiven Gentransfer in bestimmte Zell- oder Gewebetypen des Menschen oder anderer Säuger, weil das den Eintritt amphotroper retroviraler Vektoren und den Gentransfer vermittelnde Empfängerprotein (Rezeptor) für die MLV-Hüllproteine auf der Oberfläche der Säugerzellen auf fast allen diesen Zellen zu finden ist.

Im Rahmen der Gentherapie steht zur Zeit die stabile Übertragung unterschiedlicher Gene in Zellkultur, d. h. "*ex vivo*", im Vordergrund. Verbesserungen der retroviralen Vektoren wurden bisher erreicht, indem statt des retroviralen *env*-Gens des MLV die *env*-Gene anderer Retroviren in die Verpackungszelle eingebracht wurden. Beispielsweise wurden statt des MLV *env*-Gens die *env*-Gene des G-Proteins des "vesicular stomatitis virus (VSV)" (Burns et al., *Proc. Natl. Acad*. *Sci. USA* 90 (1993), 8033-8037) benutzt. Die resultierenden retroviralen Vektoren zeigten unter anderem eine erhöhte Stabilität. In der WO 96/17071 sind retrovirale Vektoren beschrieben, die statt des MLV *env*-Gens das *env*-Gen des "human spumaretrovirus (HSRV)" enthalten. HSRV zeigt jedoch wie amphotropes MLV keinerlei Spezifität bei der Infektion von Zellen. Alle bisher untersuchten Säugerzellen sind, unabhängig vom Spenderorganismus oder dem Zelltyp, permissiv für eine Infektion mit HSRV (Schweizer et al, *J. Virol.* 71 (1997), 4821-4824, und Russel et al., *J. Virol.* 70 (1996), 217-222). Auch der mögliche Einsatz der *env-*Gene des "simian sarcoma associated virus" (Takeuchi et al., *Virology* 186 (1992), 792-794), des "feline leukemia virus, subgroup B" (Porter et al., *Hum. Gene Ther.* 7 (1996), 913-919), des "feline endogenous virus RD114" (Cosset et al., *J*. *Virol.* 69 (1995), 7430-7436) und des "human T-cell leukemia virus I (HTLV-I)" (Vile et al., *Virology* 180 (1991), 420-424) deutete sich in bestimmten Experimenten an. Versuche zur Herstellung retroviraler Vektoren, welche das *env*-Gen der Lentiviren HIV-1, HIV-2 oder "simian immunodeficiency virus (SIV)" enthielten, waren bisher nicht erfolgreich. Derartige retrovirale Vektoren würden die Kernproteine, codiert von der *gag*-Region des MLV, und die Hüllproteine, codiert von der *env*-Region eines anderen Retrovirus, beispielsweise des HIV oder SIV, enthalten.

Für den selektiven Gentransfer in CD4-positive Säugerzellen stehen bisher keine Vektoren zur Verfügung. Der vorliegenden Erfindung liegt deshalb die Aufgabe zu Grunde, retrovirale Vektoren in Formen bereitzustellen, die nicht den amphotropen Rezeptor der Säugerzellen, sondern andere, nur in bestimmten Geweben und Zelltypen exprimierte Rezeptoren ansteuern. Diese Vektoren sind für eine spezifische Genübertragung in ausgewählte Zelltypen von Säugern geeignet. Eine weitere Aufgabe ist es, ein Verfahren zur Herstellung solcher retroviralen Vektoren zu entwickeln.

Diese Aufgaben werden erfindungsgemäß durch die Bereitstellung retroviraler Vektoren umfassend Viruskerne und Virushüllen gelöst, die dadurch gekennzeichnet sind, daß die Viruskerne vom murinen Leukämievirus (MLV) und die Virushüllen von menschlichen Immunschwächeviren (HIV) oder Affen-Immunschwächeviren (SIV) stammen. Insbesondere sind die retroviralen Vektoren dadurch gekennzeichnet, daß die Virushüllen vom menschlichen Immunschwächevirus 1 oder 2 (HIV-1 bzw. HIV-2) oder vom Affen-Immunschwächevirus [z.B. *Cerecopithecus aethiops* (SIVagm), *Macaca mulatta* (SIVmac), *Pan troglodydytes* (SIVcpz), *Cerecopithecus mitis* (SIVsyk), *Papio sphinx* (SIVmnd), *Cercocebus atys* (SIVsm) oder *Macaca nemestrina* (SIVmne)] stammen. Besonders bevorzugt sind retrovirale Vektoren, deren Virushüllen das Vollängen-Oberflächenprotein und eine verkürzte Form des transmembranen Hüllproteins enthalten. Insbesondere bevorzugt sind retrovirale Vektoren, deren Virushüllen das Vollängen-Oberflächenprotein und eine verkürzte Form des transmembranen Hüllproteins enthalten, das durch den C-Terminus oder ein beliebiges Fragment des transmembranen Proteins des murinen Leukämievirus (MLV) oder eines anderen Retrovirus verlängert worden ist.

Weiterhin werden Verpackungszellen bereitgestellt, in denen das psi-negative Hüllprotein-Gen (*env*) der Lentiviren HIV oder SIV und die psi-negativen *gag/pol*-Gene des MLV exprimiert werden. Diese Verpackungszellen enthalten weiterhin psi-positive Expressionskonstrukte, welche mit Hilfe der erfindungsgemäßen retroviralen Vektoren übertragen werden.

In einer Ausführungsform der Erfindung können Viruskerne, die von einem definierten Retrovirus stammen, im Zusammenhang mit Expressionskonstrukten zur Herstellung der retroviralen Vektoren benutzt werden, die aufgrund ihrer Eigenschaften, hier aufgrund des Vorhandenseins des Verpackungssignals psi, in die Viruskerne aufgenommen werden. Die Viruskerne mit den zu übertragenden Expressionskonstrukten werden ummantelt von fremden Virushüllen, die von einem anderen Virustyp oder aus einer anderen Zelle stammen. Die Expressionskonstrukte werden dann mit Hilfe der retroviralen Vektoren übertragen. Der Einbau der fremden Virushülle kann z.B. durch die Verwendung der vorzugsweise verkürzten Form des transmembranen Hüllproteins des HIV-1 *env*-Gens pTr712 vermittelt werden. Ferner kann der Einbau der fremden Virushülle z.B. durch die Verwendung der verkürzten Form des transmembranen Hüllproteins des SIVagm3 *env*-Gens *Δ0env* vermittelt werden. In einer weiteren Ausführungsform der Erfindung können Vollängen-Transmembranproteine oder modifizierte Transmembranproteine durch Anhängen des C-Terminus des transmembranen Hüllproteins des MLV oder der C-Termini der transmembranen Hüllproteine andere Viren an die verkürzten oder an die Vollängen-Transmembranproteine verwendet werden. Diese modifizierten Hüllproteine können in Vektoren aufgenommen werden. Besonders bevorzugt für solche Vektoren sind von MLV abgeleitete Kapsidpartikel, welche die Hüllproteine anderer Retroviren, insbesondere anderer Lentiviren wie HIV oder SIV enthalten. Diese Vektoren infizieren den gewünschten Zelltyp über den zellulären Rezeptor des Virus, von welchem die fremde Hülle abstammt.

In einer weiteren erfindungsgemäßen Ausführungsform wird eine beliebige Zelle mit einem psi-negativen Expressionsgen für *gag-* und *pol*-Gene transfiziert. Ferner kann die Zelle mit einem Expressionskonstrukt, umfassend ein psi-Verpackungssignal und die in eine Zielzelle zu überführende genetische Information, transfiziert werden. Die Zelle wird dann mit einem weiteren Expressionsgen transfiziert, das die genetische Information für fremde Hüllproteine enthält. Die so hergestellte Zellinie produziert retrovirale Vektoren, die die zu überführende genetische Information enthalten.

In einer bevorzugten Ausführungsform wird die MLV-*env*-negative Verpackungszellinie TELCeB6 (Cosset et al., *J*. *Virol.* 69 (1995), 7430-7436) mit dem verkürzten HIV-1 *env*-Gen pTr712 des Plasmids pLβAc/*env*-Tr712-neo (Wilk et al., *Virology.* 189 (1992), 167-177; Kräusslich et al., *Virology* 192 (1993), 605-617) transfiziert. Dabei entsteht eine Verpackungszellinie, die die erfindungsgemäßen retroviralen Vektoren des Typs MLV(HIV-1) in den Zellkulturüberstand abgibt. Diese Vektoren enthalten Kapsidpartikel, die aufgrund der Expression des *gag/pol*-Gens des MLV in der Zelle entstehen, sowie Hüllproteine des HIV-1, welche durch die intrazelluläre Expression der verkürzten Fassung Tr712 des HIV *env*-Gens in die Vektorpartikel aufgenommen werden. Die erfindungsgemäßen Vektoren enthalten das Vollängen-Oberflächenprotein gp 120-SU des HIV-1 und die verkürzte Form des transmembranen Hüllproteins, dessen kodierender Anteil aufgrund eines Stopkodons (Position 712) entsteht. Im einzelnen wurde nachgewiesen, daß dies zur Herstellung retroviraler Vektoren für den selektiven Gentransfer in CD4-positive Säugerzellen führte. CD4-positive, nicht jedoch CD4-negative Zellen zweier ansonsten identischer Linien konnten mit den Vektoren spezifisch transduziert werden, d. h. das Expressionskonstrukt-Gen wurde überführt. Die Transduktion wurde in Gegenwart von Antikörpern gehemmt, welche die Infektion mit HIV ebenfalls hemmen. Das Oberflächenhüllprotein des HIV wurde auf der Oberfläche der hergestellten Gentransfervektoren nachgewiesen.

In einer weiteren bevorzugten Ausführungsform wird die MLV-*env*-negative Verpackungszellinie TELCeB6 mit dem verkürtzten SIVagm3 *env*-Gen *Δ0env* transfiziert. Die erhaltene Verpackungszellinie sezerniert die erfindungsgemäßen retroviralen Vektoren des Typs MLV(SIVagm3) in den Zellkulturüberstand. Diese Vektoren enthalten ebenfalls Kapsidpartikel des MLV und Hüllproteine des SIVagm3, welche durch die intrazelluläre Expression der verkürzten Fassung *Δ0env* des SIVagm3 *env*-Gens in die Vektorpartikel aufgenommen werden.

Die vorliegende Erfindung eröffnet somit die folgenden Möglichkeiten:
- Gene in CD4-positive Säugerzellen selektiv zu überführen,
- weitere Effizienzsteigerungen des Gentransfers durch Verbesserung der env-Genkonstrukte in vergleichbaren Verpackungszellen zu erreichen,
- Gentherapiestrategien zu entwickeln, für die ein selektiver Gentransfer in CD4-positive Säugerzellen notwendig oder hilfreich erscheint,
- insbesondere Gentherapiestrategien zur Behandlung oder Prophylaxe der HIV-Infektion des Menschen zu entwickeln, wobei HIV-hemmende Gene, z.B. Antisensegene, RNA-Ködergene oder transdominant-negative mutante Gene des HIV oder anderer Lentiviren in CD4-positive Zellen überführt werden,
- insbesondere Gentherapiestrategien zur Einführung von Genen in CD4-positive Zellen zu entwickeln, zur Behandlung oder Prophylaxe von Erbkrankheiten, wie z.B. der ADA-Defizienz oder anderen genetisch behandelbaren Krankheiten, bei denen die Einführung in bestimmte Zellen vorteilhaft ist, und
- den Eintritt von Lentiviren in Säugerzellen im Detail zu untersuchen.

Die Abbildungen dienen der Erläuterung der Erfindung.

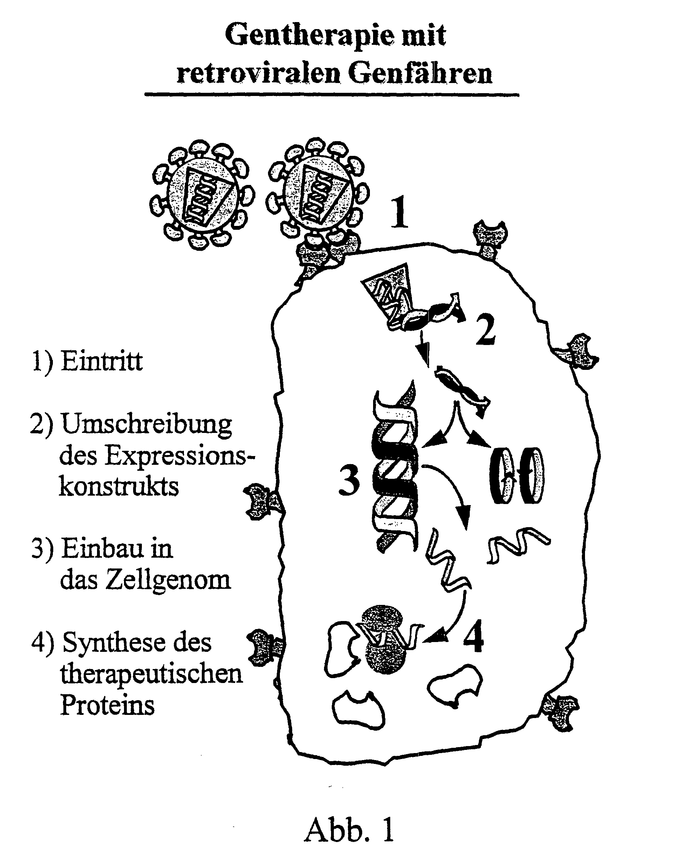

Abbildung 1 zeigt schematisch das Prinzip der Übertragung von Genen mit Hilfe retroviraler Vektoren in Säugerzellen.

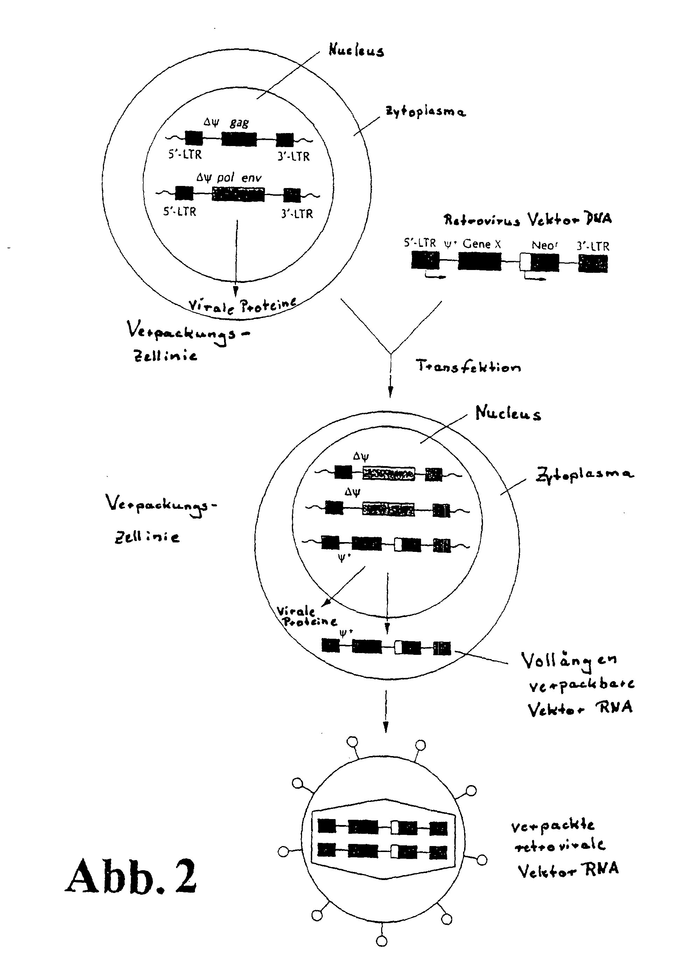

Abbildung 2 zeigt ein Schema der Herstellung eines retroviralen Vektors (entnommen aus "Molecular Biotechnology", Principles and Applications of Recombinant DNA, B.R. Glick und J.J. Pasternak, ASM Press, Washington, D.C., 1994, Seite 411, und ins Deutsche übertragen).

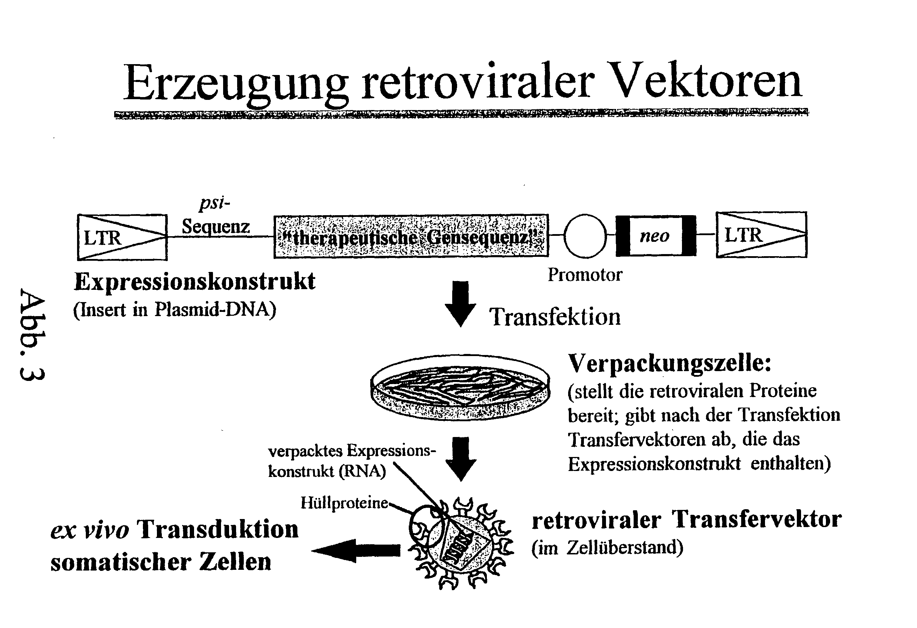

Abbildung 3 zeigt schematisch einen allgemein anwendbaren Herstellungsweg für retrovirale Vektoren (retrovirale Transfervektoren) durch Transfektion einer zunächst Expressionskonstruktnegativen Verpackungszelle mit einem Expressionskonstrukt, das aufgrund des Vorhandenseins des Verpackungssignals (hier: psi) das zu übertragende Gen (therapeutische Gensequenz) in die retroviralen Vektoren einschleust.

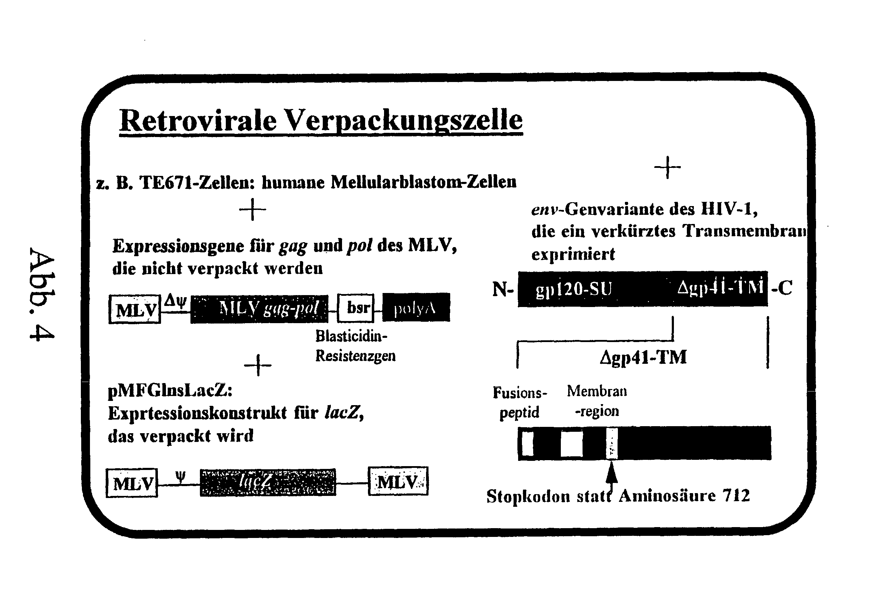

Abbildung 4 zeigt einen speziellen Herstellungsweg für retrovirale Vektoren des Typs MLV(HIV-1). Dargestellt sind das MLV *gag*/*pol*-Expressions-Gen und das psi-positive Expressionskonstrukt pMFGInsLacZ, welche von der MLV *env*-negativen Verpackungszelle TELCeB6 exprimiert werden. Die *env*-Genvariante des HIV-1 (Tr712), welche die für das Vollängenoberflächen-Hüllprotein gp120-SU und die verkürzte Version des transmembranen Hüllproteins (Δgp41-TM) kodierenden Regionen enthält, wird bei der Herstellung der MLV(HIV-1)-Vektoren ebenfalls in dieser Zelle exprimiert.

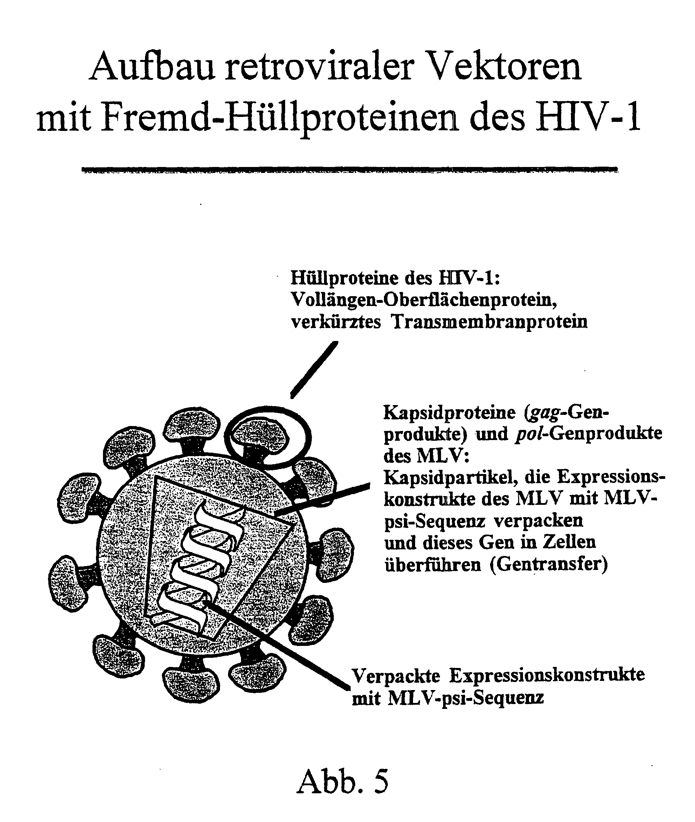

Abbildung 5 zeigt den generellen Aufbau von retroviralen Vektoren, welche den Viruskern eines bestimmten Virus in Kombination mit der Virushülle eines anderen Virus enthalten, erläutert am Beispiel der MLV(HIV-1)-Vektoren.

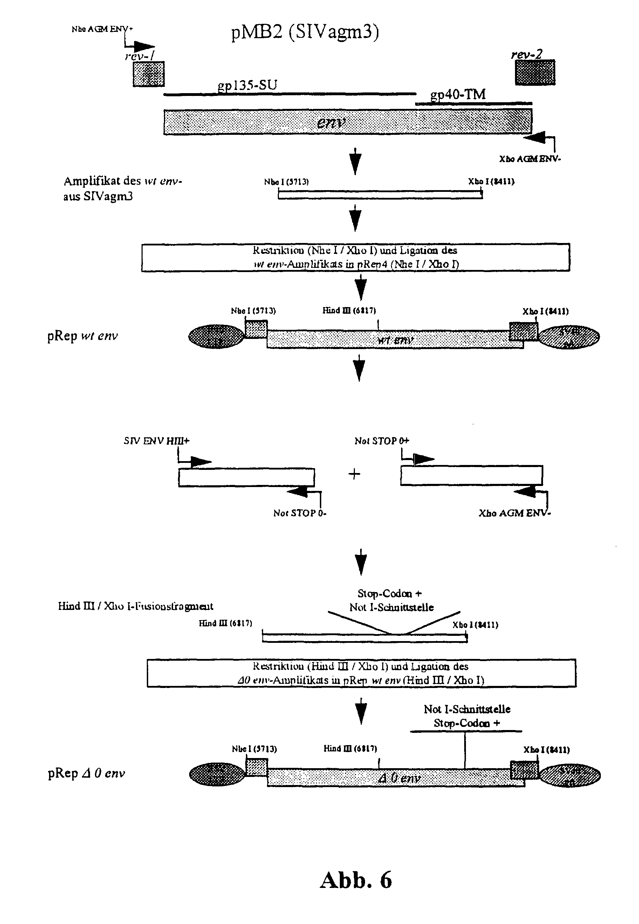

Abbildung 6 zeigt schematisch das Klonierungsschema zur Klonierungsstrategie des SIVagm3 *env*-Expressionskonstrukts pRep *wt env* und der verkürzten *env*-Gene, erläutert am Beispiel der Variante *Δ0 env.*

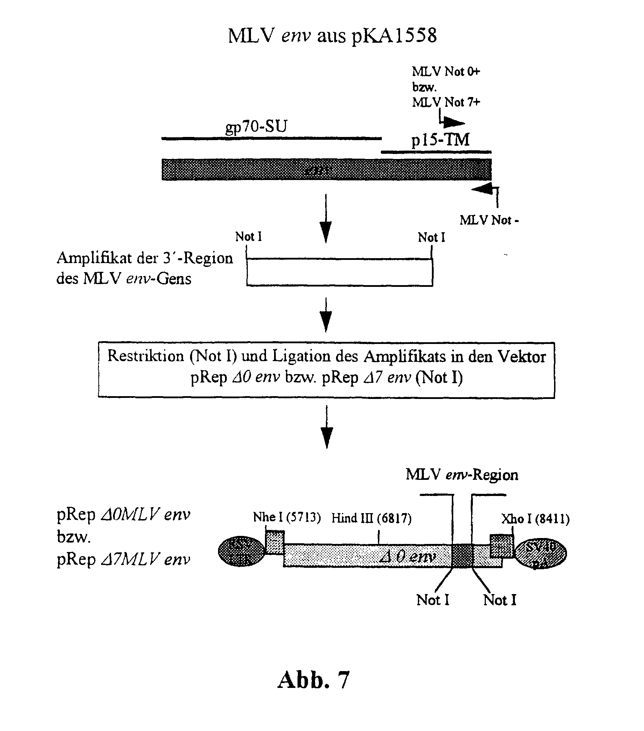

Abbildung 7 zeigt schematisch das Klonierungsschema zur Klonierungsstrategie der SIVagm3 *env*-Fusionsgene *Δ0MLV env* und *Δ7MLV env.*

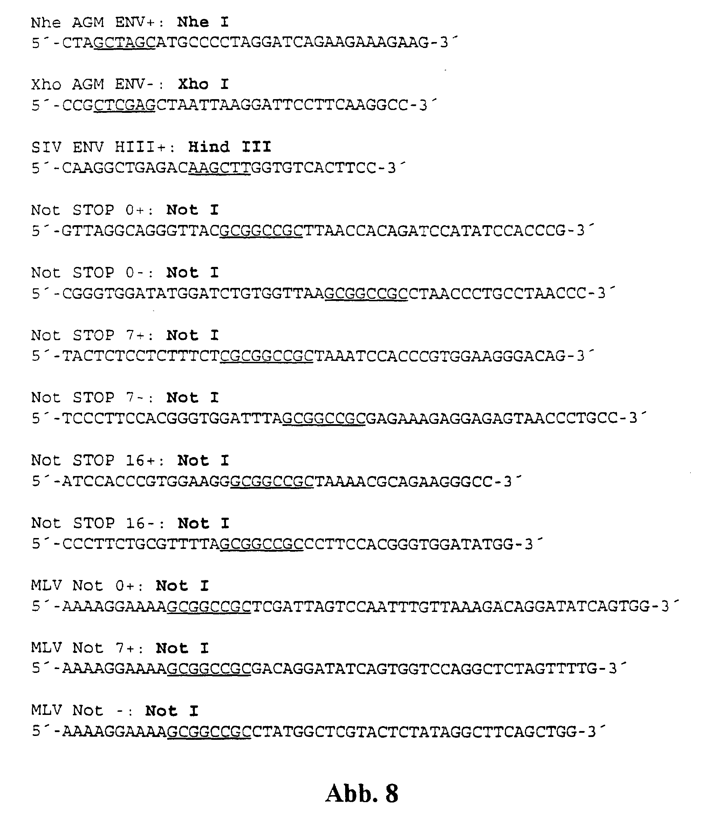

Abbildung 8 zeigt die Nukleinsäuresequenzen der zur Klonierung der SIVagm3 env-Expressionskonstrukte verwendeten Oligonukleotide. Die Nukleinsäuresequenzen der Restriktionsschnittstellen sind unterstrichen.

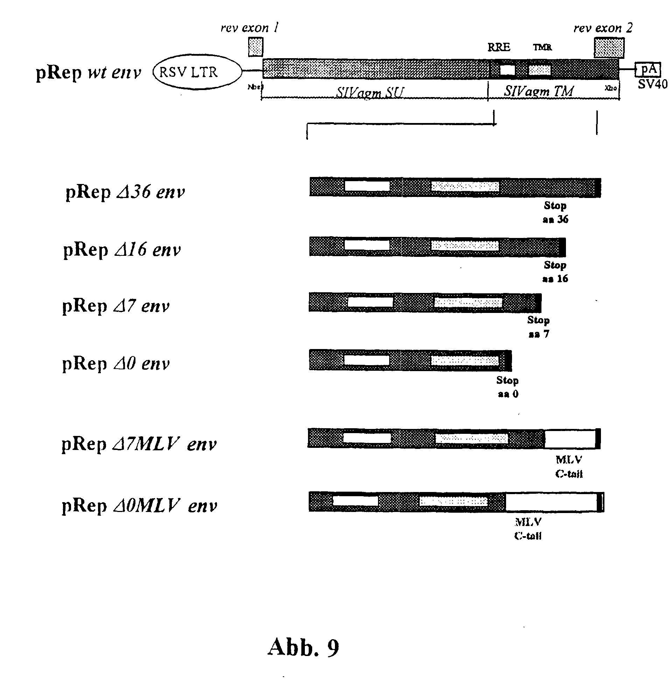

Abbildung 9 zeigt schematisch die transkriptionellen Einheiten der SIVagm3 *env*-Konstrukte.

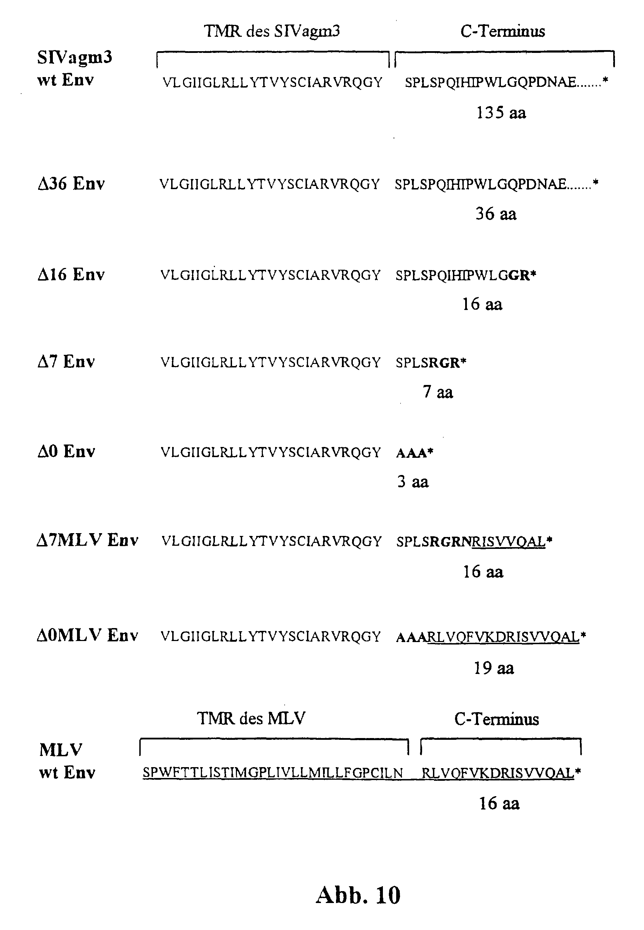

Abbildung 10 zeigt die Aminosäuresequenzen der intrazellulären Domänen der Genprodukte von SIVagm3 *env*-Konstrukten. Zum Vergleich sind die Sequenzen des SIVagm3 und des MLV angegeben. Die Aminosäuren sind im Einbuchstabencode dargestellt. Aminosäuren, die von MLV-Sequenzen abgeleitet wurden, sind unterstrichen. Die in den Namen der Konstrukte enthaltenden Zahlen beziehen sich auf die N-terminal verbliebenen Aminosäuren nach der Transmembranregion und vor den mittels rekombinanter PCR inserierten Stopcodons. Durch die Insertion einer Not I-Restriktionsschnittstelle folgen allerdings je zwei oder drei Aminosäuren, welche nicht in der nativen Sequenz des SIVagm3 vorkommen. Die durch die inserierten Not I-Schnittstellen kodierten Aminosäuren sind fett gedruckt dargestellt. "......" steht für Aminosäuren des SIVagm3, die nicht im einzelnen aufgeführt sind. "*" kennzeichnet den C-Terminus der Proteine. Die Länge der intrazellulären Domänen ist angegeben. "aa" bedeutet Aminosäuren. "TMR" bedeutet Transmembranregion. Die Bezeichnung "*MLV*" steht für die inserierten 3'-liegenden Sequenzen des MLV *env*-Gens. Die inserierten C-Termini des MLV beinhalten jeweils das sogenannte p2-Protein (bestehend aus 16 aa), welches intrazellulär durch Proteolyse abgespalten wird, bevor die Hüllproteine in die Virionen eingebaut werden.

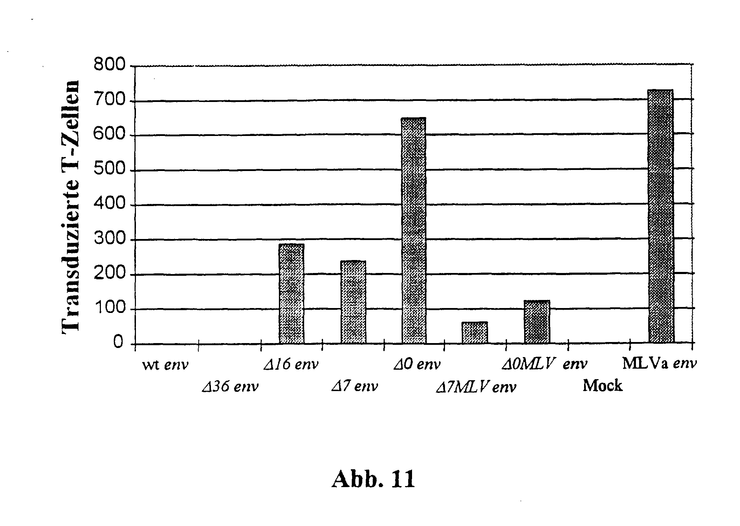

Abbildung 11 ist eine graphische Darstellung der Effizienz der Bildung von MLV(SIVagm)-Vektoren nach Transfektion der *env*-Varianten in TELCeB6/*rev*-Zellen. Die T-Zellen wurden für zwei Tage im selben Medium und in Gegenwart der Transfektanden kokultiviert und anschließend mittels X-Gal-Test auf den erfolgten Gentransfer hin untersucht. Die angegebenen Werte stellen Mittelswerte aus zwei Experimenten dar. "Mock bezeichnet die Negativkontrolle.

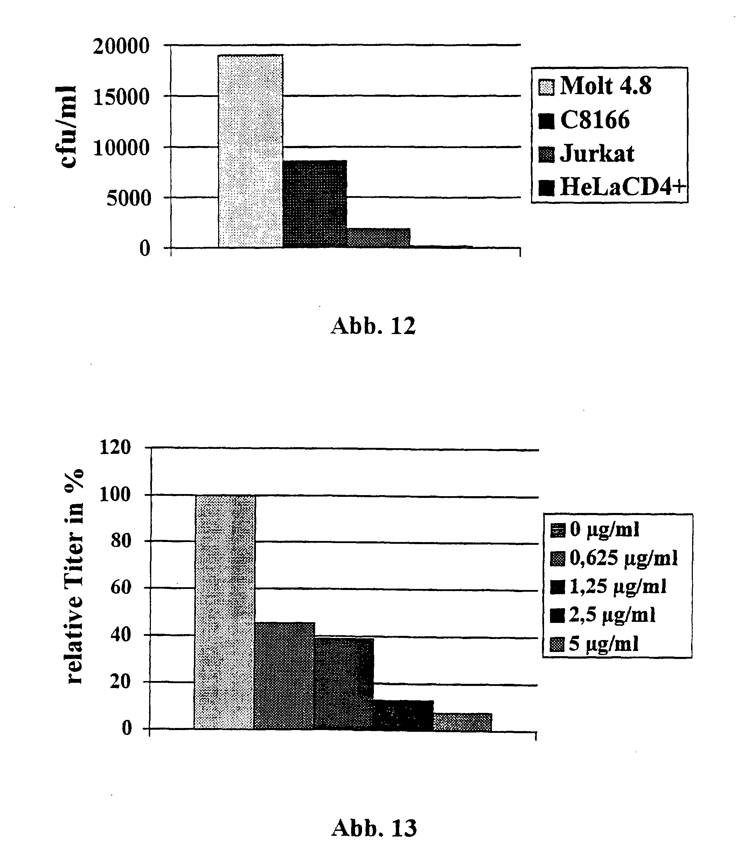

Abbildung 12 ist eine graphische Darstellung der Ergebnisse der Titration der MLV(SIVagm)-Vektorstocks auf verschiedenen CD4⁺-Zellinien.

Abbildung 13 ist eine graphische Darstellung der Ergebnisse der Inhibition der Transduktion von T-Zellen mittels Blockade des zellulären CD4-Rezeptors durch monoklonale anti-CD4 Antikörper. Die eingesetzten Konzentrationen des Antikörpers IOT4a sind in der Legende angegeben. Die erreichten Transduktionseffizienzen in Abwesenheit der Antikörper wurden als Referenz 100% gleichgesetzt und die Titer in Anwesenheit der Antikörper in Prozent dieser Positivkontrolle ausgedrückt.

Der hier verwendete Begriff retroviraler Vektor bedeutet ein replikationsdefizientes retrovirales Viruspartikel, das anstelle der retroviralen mRNA eine fremde eingeführte RNA eines Gens, z.B. eines therapeutischen Gens oder dessen Fragment oder eines Reportergens übertragen kann. Der hier verwendete Begriff "therapeutisches Gen" bedeutet eine Nukleinsäuresequenz, die in die Zielzelle mittels des retroviralen Vektors eingeführt werden soll und umfaßt komplette Gene, deren Fragmente, Antisense-Nukleinsäuren und dergleichen. Der hier verwendete Begriff SIV bedeutet Viren der Familie des Affen-Immunschwächevirus, z.B. *Cerecopithecus aethiops* (SIVagm), *Macaca mulatta* (SIVmac), *Pan troglodydytes* (SIVcpz), *Cerecopithecus mitis* (SIVsyk), *Papio sphinx* (SIVmnd), *Cercocebus atys* (SIVsm) oder *Macaca nemestrina* (SIVmne). Der hier verwendete Begriff SIVagm3 bezeichnet den Molekularklon SIVagm3mc (Baier et al., *J*. *Virol.* 62 (1989), 4123-4128).

Die Herstellung von MLV(HIV-1)- und MLV(SIVagm3)-Vektoren der Erfindung wird nachfolgend näher erläutert.

Zunächst wird eine DNA-Sequenz hergestellt, welche die Bildung der notwendigen Proteine induziert, die zur Bildung von Viruskernen in der Lage sind. Die DNA-Sequenz wird in eine humane Wirtszelle transferiert und dort exprimiert. Die DNA-Sequenz enthält zusätzlich Operator-Elemente, die zur Expression der DNA-Sequenz erforderlich sind, welche die Bildung der Viruskerne induzieren. In die so gewonnene Wirtszelle wird eine zweite DNA-Sequenz übertragen, die zur Bildung von Hüllproteinen führt, die nicht von dem Virus stammen, von welchem die Viruskerne stammen. Danach wird eine weitere DNA-Sequenz eingebracht, die von den Viruskernen verpackt wird und Sequenzen enthält, die zur Bildung der therapeutischen Gensequenzen oder der gewünschten Proteine in der Zelle führen, in die mit Hilfe des retroviralen Vektors therapeutische Gene übertragen werden sollen.

Die DNA-Sequenz, welche zur Expression der Vektorhüllproteine führt, kann vorzugsweise vom *env*-Gen des HIV-1, HIV-2 oder anderer HIV-Stämme, des SIV, z.B. *Cerecopithecus aethiops* (SIVagm), *Macaca mulatta* (SIVmac), *Pan troglodydytes* (SIVcpz), *Cerecopithecus mitis* (SIVsyk), *Papio sphinx* (SIVmnd), *Cercocebus atys* (SIVsm) oder *Macaca nemestrina* (SIVmne) stammen. Die verwendeten *env*-Gene können der Originalversion der genannten Viren entsprechen oder verkürzte Formen dieser *env*-Gene oder sogar modifizierte Formen dieser *env*-Gene sein. Das besonders bevorzugte HIV-1 *env*-Gen-pTr712 führt zur Bildung eines Vollängen-Oberflächenproteins gp120-SU und eines verkürzten Transmembranproteins. Das ebenfalls besonders bevorzugte SIVagm3 *env*-Gen *Δ0env* führt zur Bildung eines Vollängen-Oberflächenproteins gp130-SU und eines verkürzten Transmembranproteins. Das Transmembranprotein kann auch z. B. durch das Anhängen der C-terminalen Domäne oder eines beliebigen anderen Fragments des Transmembranproteins des MLV oder eines anderen Virus modifiziert sein. Beispielsweise kann bei Verwendung von MLV Kapsidpartikeln der C-Terminus des Transmembranproteins des MLV anstelle des C-Terminus des HIV-1 *env*-Gens oder SIVagm *env*-Gens verwendet werden. Dabei kann die Spaltstelle des C-terminalen p2 Peptids modifiziert sein oder nicht.

Die genannten erfindungsgemäßen viralen Vektoren können zur Übertragung von therapeutischen Genen in bestimmte Zelltypen verwendet werden. Beispielsweise übertragen die erfindungsgemäßen MLV(HIV-1)- und MLV(SIVagm3)-Vektoren Gene speziell in CD4-positive Zellen. Diese Vektoren besitzen somit den Tropismus des HIV-1 bzw. SIVagm3, deren Hüllproteine sie enthalten.

Die nachfolgenden Beispiele erläutern die Erfindung und sind nicht einschränkend zu verstehen.

### Beispiel 1: Herstellung von MLV(HIV-1)-Vektoren

### 1.1 Zellinien und Plasmide

Alle verwendeten Plasmide wurden aus transformierten *E*. *coli* der bekannten Stämme DH10α oder HB101 präpariert. Die molekulare Klonierung der Expressionskonstrukte pLβAc/*env*-neo ist von Kräusslich et al., *Virology* 192 (1993), 605-617 und pLssAc/*env*-Tr712-neo ist von Kräusslich et al., *Virology* 192 (1993), 605-617 und Wilk et al., *Virology.* 189 (1992), 167-177 beschrieben. Diese Expressionskonstrukte kodieren die HIV-1 *env*-Gen-Varianten und das Neomycin-Resistenzgen. Das Expressionskonstrukt pCRUCA, welches das *env*-Gen des amphotropen MLV umfaßt, wurde von Wilk et al., *Virology.* 189 (1992), 167-177 und Battini et al., *J*. *Virol.* 66 (1992), 1468-1475 beschrieben. Die TELCeB6 Verpackungszellinie, die das retrovirale Expressionskonstrukt MFG-nlsLacZ sowie die Gene *gag* und *pol* des MLV exprimiert, wurde von Cosset et al., *J. Virol*. 69 (1995), 7430-7436 beschrieben. Die HeLa-CD4+ Zellen wurden über das MRC AIDS Programm Reagents Project bezogen, die 293-Zellen wurden von ATCC (ATCC CRL 1573) bezogen. Alle adhärenten Zellen wurden in Dulbecco's Modified Eagles Medium (GIBCO/BRL, Eggenstein, Deutschland) in Gegenwart von 10 % fetalem Kälberserum gehalten. Die humane T-Zellinie Molt 4 (ATCC CRL 1582)wurde in RPMI-1640-Medium mit 10 % fetalem Kälberserum (GIBCO/BRL, Eggenstein, Deutschland) propagiert. Die Transfektion der Verpackungszellinie TELCeB6 mit dem Expressionskonstrukt pTr712 wurde mittels Lipofektamin (GIBCO/BRL, Eggenstein, Deutschland) gemäß den Angaben des Herstellers durchgeführt. Nach Transfektion des Plasmids pREP4 (Invitrogen, Leek, Niederlande) wurde die Hygromycin-Selektion in Gegenwart von 200 mg /ml Hygromycin B (Sigma, Deisenhofen, Deutschland) durchgeführt. Einer der daraus hervorgegangenen Klone stellt die Zellinie TELCeB6/pTr712-K 14 dar.

### 1.2 Virale Infektion, Bestimmung von Titern und Neutralisationsexperimente

Die adhärenten Zellen wurden in 24-Well(Vertiefung)-Platten in einer Dichte von 4 x 10⁴ Zellen pro Well oder in 6-Well-Platten in einer Dichte von 2 x 10⁵ Zellen pro Well ausgesäht. Die Molt-4-Zellen wurden in 6-Well-Platten in einer Dichte von 8 x 10⁵ ausgesäht. Vor der Infektion wurden die Zellen über Nacht in Zellmedium inkubiert. Zur Infektionen wurden die Zielzellen drei Stunden mit 1 ml verdünnten oder unverdünnten Retroviruspartikel-enthaltenden Überständen koinkubiert. Die Virionen enthaltenden Überstände wurden vorher durch einen 0,45 mm-Filter passagiert, um die kontaminierenden Zellen zu entfernen. Zwei Tage nach der Infektion wurden die Zielzellen mittels x-Gal-Test auf β-Gal-Expression untersucht. Die viralen Titer wurden wie beschrieben bestimmt. Die Titer sind in Kolonie bildenden Einheiten pro ml angegeben (*colony forming units,* cfu). Zur Neutralisation der pseudotypisierten Vektoren wurde Serum eines HIV-1 infizierten Spenders eingesetzt.

### 1.3 Immunanfärbung der Tranfektanden

Die mit der DNA der Plasmide, welche die Hüllproteine des HIV-1 kodierten, transfizierten TELCeB6-Zellen wurden mit PBS gewaschen und anschließend 15 min mit eiskaltem Methanol inkubiert. Nach wiederholtem Waschen wurde eine Stunde lang Blockierungspuffer (PBS/ 2 % BSA) zu den Zellen gegeben. Die Zellen wurden erneut gewaschen und anschliessend mit einer 1:1000 verdünnten anti-HIV-1 Serumlösung 1 Stunde inkubiert. Nach wiederholtem Waschen wurden die Zellen mit Peroxidase-gekoppeltem Protein G (Bio-Rad, Krefeld, Deutschland) inkubiert. Schließlich wurden die Antigen-präsentierenden Zellen durch Zugabe von Substratpuffer (H₂0₂ mit 3-Amino-9-ethylcarbazol, Sigma, Deisenhofen, Deutschland) angefärbt. Es konnte gezeigt werden, daß das Plasmid pTr712 die Bildung der von HIV-1 abgeleiteten Hüllproteine in den Transfektanden erlaubte.

### 1.4 Western-Blot-Analyse

Die Herstellung der Zellysate und die Durchführung der Western-Blots erfolgte nach üblichen Verfahren. Die Viruspartikel in den Überständen der mit den von HIV-1 abgeleiteten Hüllproteinen kodierenden Plasmiden transfizierten Verpackungszellen wurden mittels Ultrazentrifugation (45 min bei 200.000 x g und 40°C) aufkonzentriert. Die Zentrifugate wurden in Probenpuffer aufgenommen und mittels SDS-Polyacrylamid-Gelelektrophorese aufgetrennt. Die Western-Blot-Analyse wurde unter der Verwendung eines Ziegenserums gegen HIV-1 gp 120-SU und Peroxidase-gekoppeltem Protein G durchgeführt. Die Proteinbanden wurden mittels ECL-Detektionskit (Amersham, Braunschweig, Deutschland) nachgewiesen. Sowohl in den Zellysaten als auch in den Vektorpartikeln konnte das Oberflächen-Hüllprotein gp120-SU des HIV-1 nachgewiesen werden.

### 1.5 Membranfusionseigenschaft des HIV-1-Hüllproteins

Eine subkonfluente Kultur der Verpackungszellinie TELCeB6/pTr712-K14 wurde mit Jurkat-Zellen überschichtet, 48 h propagiert und anschließend photographiert. Es wurde eine Reihe von Synzytien beobachtet, so daß die Funktionalität der von der obengenannten Zellinie gebildeten Hüllproteine des HIV-1 gezeigt werden konnte.

### Beispiel 2: Herstellung von MLV(SIVagm3) Vektoren

### 2.1 Klonierung der SIVagm3 env-Expressionskonstrukte

Zur Klonierung rekombinanter Varianten des *env*-Gens des SIVagm3 wurde das Plasmid pRep 4 (Invitrogen, Leek, Niederlande) verwendet. Zur Herstellung der *env*-Varianten wurde zunächst die Sequenz zwischen den Basen 5713 und 8411 des SIVagm3 aus dem Plasmid pMB2 (Baier et al., *J*. *Virol.* 63 (1989), 5119-5123) mittels rekombinanter PCR (rPCR) unter Verwendung der Oligonukleotide Nhe AGM ENV+ (SEQ ID NO:1) und Xho AGM ENV- (SEQ ID NO:2) amplifiziert und über die Restriktionsschnittstellen Nhe I und Xho I in den Vektor pRep4 inseriert. Die mittels rPCR hergestellte cDNA umfaßt beide *rev*-Exons und den gesamten Leserahmen des *env*-Gens. Das erhaltene Plasmid wurde pRep *wt env* genannt und diente im folgenden als Matrize für weitere rPCRs und als Vektor für weitere Klonierungen.

Zunächst wurden die verkürzten *env*-Gene *Δ0 env*, *Δ7 env* un *Δ16 env* kloniert. Dazu wurden zunächst die jeweiligen Oligonukleotide Not STOP 0-/+ (0-: SEQ ID NO:3, 0+: SEQ ID NO:4), Not STOP 7-/+ (7-: SEQ ID NO:5, 7+: SEQ ID NO:6) und Not STOP16-/+ (16-: SEQ ID NO:7, 16+: SEQ ID NO:8), in PCRs eingesetzt, um das gewünschte Stop-Codon und eine 5' liegende Not I-Restriktionsschnittstelle zu inserieren. Als flankierende Oligonukleotide dienten jeweils die Oligonukleotide SIV ENV HIII+ (SEQ ID NO:9) und Xho AGM ENV- (SEQ ID NO:2), um die Amplifikate möglichst klein und somit die Wahrscheinlichkeit der fehlerhaften Amplifikation durch die *Taq*-Polymerase gering zuhalten. Die in den einzelnen PCRs erhaltenen Amplifikate wurden in üblicher Weise isoliert und dann in Fusions-PCR eingesetzt (Amplifikat SEQ ID NO:9/3 mit Amplifikat SEQ ID NO:4/2; Amplifikat SEQ ID NO:9/5 mit Amplifikat SEQ ID NO:6/2; Amplifikat SEQ ID NO:9/7 mit Amplifikat SEQ ID NO:8/2). Das erste Amplifikat enthält dabei die Sequenzen des SIVagm *env* von der Hind III-Schnittstelle (6817) bis zum-Primer (Not STOP 0- / 7- / 16-), welcher eine NOT I-Schnittstelle und das zu inserierende Stopcodon sowie die in der Matrize 3'-folgenden Sequenzen enthält. Das zweite Amplifikat beginnt 5'mit den +Primern (Not STOP 0+ / 7+ / 16+), welche die letzten 3'-liegenden Basen des ersten Amplifikates, eine Not I-Schnittstelle und das Stopcodon enthält, und endet mit der Sequenz des Xho AGM ENV-Primers. Die in der Fusions-PCR jeweils eingesetzten Amplifikate besaßen somit überlappende Sequenzen, die eine Hybridisierung zuließen. Mittels Fusions-PCR wurden die aneinander hybridisierten Amplifikate unter Verwendung der Primer Xho AGM ENV- und SIV ENV HIII+ amplifiziert. Die so erhaltenen Fusionsfragmente wurden nach Restriktion (Hind III / Xho I) in den Vektor Rep *wt env* (Hind III / Xho I) kloniert. Die *env*-Varianten *Δ0MLV env* und *Δ7MLV env* wurden aus den Varianten *Δ0env* und *Δ7env* hergestellt.. Hierzu wurden die 3'-liegenden Regionen des MLV *env*-Gens, welche die intrazellulären Anteile des TM-Proteins p15 codieren, mittels rPCR unter Verwendung der Oligonukleotide MLV Not(SEQ ID NO:10) und MLV Not 7+ (SEQ ID NO:11) bzw. MLV Not 0+ (SEQ ID NO:12) amplifiziert. Der molekular Klon pKA1558 wurde von Scov et al., *J*. *Gen. Virol.* 74 (1993), 707-714 beschrieben und diente hier als Matrize. Die Amplifikate wurden über die Restiktionsschnittstelle Not I in die *env*-Varianten *Δ0env* und *Δ7env* inseriert.

### 2.2 Polymerase-Ketten-Reaktion (PCR)

Zur Amplifizierung der gewünschten DNA-Abschitte wurde die Taq-DNA-Polymerase von Perkin-Elmer, Langen, Deutschland verwendet. Eine Standard-PCR (100 µl-Ansatz) enthielt: 1 x PCR-Puffer (10 mM Tris/HCl, pH 8,8, 50 mM KCl, 1,5 mM MgCl₂, 0,01% Gelatine), 10 Amplifikat SEQ ID NO:9/7 mit Amplifikat SEQ ID NO:8/2µM je Oligodesoxynukleotid-Primer, 200 µM je Desoxynukleotid (dNTP), 2,5 Einheiten Taq-Polymerase und 100 ng Plasmid-DNA. Das PCR-Programm für die Primerpaare Amplifikat SEQ ID NO:9/3 mit Amplifikat SEQ ID NO:4/2, Amplifikat SEQ ID NO:9/5 mit Amplifikat SEQ ID NO:6/2 und Amplifikat SEQ ID NO:9/7 mit Amplifikat SEQ ID NO:8/2 war wie folgt:
1. 94°C 300 sek
2. 94°C 45 sek
3. 55°C 120 sek
4. 72°C 180 + 2 sek
5. 10 Zyklen 2.-4.
6. 94°C 45 sek.
7. 60°C 120 sek.
8. 72°C 180 + 2 sek.
9. 10 Zyklen 6.-8.
10. 72°C 600 sek.

### 2.3 Zellinien, Medien, Transformation und Transfektion

Plasmide wurden in die *E.coli*-Stämme DH5α, DH10B, Top10F' (GIBCO/BRL, Eggenstein, Deutschland) und GM 2163 (Invitrogen, Leek, Niederlande) auf übliche Weise eingebracht. Die Transfektion der Verpackungszellinie TELCeB6, bzw. TELCeB6/*rev* mit den SIVagm *env*-Expressionskonstrukten wurde mittels Lipofektamin (GIBCO/BRL, Eggenstein, Deutschland) gemäß den Angaben des Herstellers durchgeführt. Die TELCeB6 Verpackungszellinie, die das retrovirale Expressionskonstrukt MFG-nlsLacZ sowie die *gag-* und *pol*-Gene des MLV exprimiert, wurde von Cosset et al., *J*. *Virol.* 69 (1995), 7430-7436 beschrieben. In die Zellinie TELCeB6/*rev* wurde ein Plasmid (pCMV-*rev*, NIH Research and Reference Reagent Program, Katalognummer 1443) durch stabile Transfektion eingebracht, welches die Expression des *rev*-Gens von HIV-1 unter der transkriptionellen Kontrolle eines CMV-Promotors erlaubte. Die Zellinie TELCeB6/*rev* exprimiert somit zusätzlich das *rev*-Gen des HIV-1. Die Zellinien TELCeB6 und TELCeB6/*rev* wurden in den nachstehend beschriebenen Medien für adhärente Zellen unter Zugabe von Glucose (4 g/L) kultiviert. Adhärente HeLa-CD4+ Zellen wurden über das MRC AIDS Programm Reagents Projekt, die HeLa-Zellen von ATCC (CCL 2) bezogen. Alle adhärenten Zellen wurden in Dulbecco's Modified Eagles Medium (GIBCO/BRL, Eggenstein, Deutschland) in Gegenwart von 10 % Komplement-inaktiviertem fetalem Kälberserum (FKS; Biochrom KG, Berlin, Deutschland), 2 mM L-Glutamin (Biochrom KG, Berlin, Deutschland) und Antibiotika (0,1 mg/ml Nystatin, 100 Einheiten/ml Penicillin, 50 mg/ml Streptomycin; Biochrom KG, Berlin, Deutschland) bei 37°C, 5% CO₂ und gesättigter Wasserdampfatmosphäre in einem Zellinkubator (Cytoperm, Heraeus, Deutschland) kultiviert. Die Suspensionszellen C8166 (humane Leukämie-T-Zellen, die das defekte HTLV-1-Genom enthalten (NIH Research and Reference Reagent Program, Katalognummer 404), Jurkat-Zellen (humane Leukämie-T-Zellen; NIH Research and Reference Reagent Program, Katalognummer 177) und Molt 4.8-Zellen (humane Leukämie-T-Zellen; NIH Research and Reference Reagent Program, Katalognummer 175) wurden in RPMI 1640 (GIBCO/BRL, Eggenstein, Deutschland) mit 10% FKS, 2 mM L-Glutamin (Biochrom KG, Berlin, Deutschland) und Antibiotika (0,1 mg/ml Nystatin, 100 Einheiten/ml Penicillin, 50 mg/ml Streptomycin; Biochrom KG, Berlin, Deutschland) kultiviert.

### 2.4 Immunperoxidase-Antiperoxidase-Test (IPAP)

Um die korrekte Expression der *env*-Varianten zu überprüfen, wurden alle *env*-Konstrukte in TELCeB6-Zellen transfiziert. Zwei Tage nach der Transfektion wurde mittels IPAP getestet, ob die Transfektanden die jeweilige *env*-Variante exprimierten. Die TELCeB6-Zellen wurden in 35 mm-Zellkulturschalen mit PBS gewaschen und mit -20°C kaltem Methanol 15 Minuten bei -20°C fixiert. Danach wurden die unspezifischen Bindungsstellen auf den erneut mit PBS gewaschenen Zellen mittels Blockierungspuffer (PBS + 2% BSA) eine Stunde bei Raumtemperatur (RT) blockiert. Anschließend wurden die Zellen mit einem SIVagm-Antiserum (aus einem mit SIVagm in üblicher Weise infizierten Schweinsaffen (*Macaca nemestrina*), das mit Blockierungspuffer verdünnt wurde, eine Stunde bei 37°C inkubiert. Nach weiteren Waschvorgängen mit PBS wurden die Fc-Anteile der Primär-Antikörper mittels Meerrettichperoxydase-konjugiertem Protein G (BIO-RAD, München, Deutschland) nach einstündiger Inkubation bei 37°C detektiert. Nach zwei Waschschritten mit PBS erfolgte die Zugabe der Substratlösung (4 mg 3-Amino-9-ethylcarbazol in 1 ml Dimethylformamid gelöst, 19 ml 20 mM NaOAc-Puffer, pH 5 und 30 µl H₂O₂; durch einen 45 µm Filter filtriert).Nach wenigen Minuten Inkubation bei RT wiesen die Virusprotein exprimierenden Zellen eine deutliche Rotfärbung auf. Bis auf die Plasmide pRep *Δ7env* und pRep *Δ7MLVenv* erlaubten alle Konstrukte die Expression der Hüllproteine des SIVagm3. Bei der Analyse der Sequenzierungsdaten wurde festgestellt, daß die beiden Varianten pRep *Δ7env* und pRep *Δ7MLVenv* ein Stopcodon im zweiten rev-Exon aufwiesen, welches durch die Insertion der Not I-Schnittstelle entstanden war. Das so unvollständig gebildete Rev-Protein war offensichtlich nicht in der Lage, den Transport der mRNA-Transkripte der *env*-Gene aus dem Zellkern zu gewährleisten.

### 2.5 Synzytieninduktion

Da die Analyse mittels IPAP nur die gebildeten viralen Hüllproteine nachweist, mußten diese auch auf ihre Funktionalität überprüft werden. Mittels Synzytieneninduktion sollte gezeigt werden, daß die Hüllproteine auf der Zellmembran präsentiert werden und Synzytien in CD4⁺-Zellen induzieren können. Dazu wurden die SIVagm *env*-Konstrukte in TELCeB6/*rev*-Zellen transfiziert. Am folgenden Tag wurden die Transfektanden mit Zellen der T-Zellinie Molt4.8 überschichtet und 24 h später fotographiert. Die auf der Zellmembran der Verpackungszellen präsentierten SIVagm-Hüllproteine sind in der Lage, die ebenfalls membranständigen CD4-Proteine der T-Zellen zu binden. So kommt es zur Verschmelzung der beiden Zelltypen. Diese Synzytien unterscheiden sich durch ihre Größe von den Parentalzellen. Alle getesteten *env*-Varianten induzierten Synzytien in diesem Test, jedoch war das Ausmaß der Synzytieninduktion sehr verschieden. Die Varianten *Δ0env* und *Δ16env* induzierten große Synzytien, während die anderen Varianten einschließlich des *wt env* nur schwache Synzytieninduktion erzeugten. Jedoch konnte für alle Varianten gezeigt werden, daß ihre Expression zur Bildung membranständiger Proteine führte, welche in der Lage waren, CD4 zu binden.

### 2.6 Nachweis von β-Galactosidase-Aktivität (X-Gal-Test)

Mittels des X-Gal-Tests wurde eine erfolgreiche Transduktion humaner Zellen mit den MLV(SIVagm)-abgeleiteten Vektoren anhand des erfolgreichen Transfers des verpackbaren Konstruktes MFGInsLacZ nachgewiesen. Der Nachweis der β-Galactosidase-Aktivität in transduzierten Zellen wurde nach einer modifizierten Methode (Sanes *et al., EMBO J. 5* (1986), 3133-3142) mittels X-Gal-Färbung erbracht. Neben dem β-Galactosidase-Substrat (5-Brom-4-chlor-3-indolyl-β-D-galactopyranosid; Sigma, Deisenhofen, Deutschland) [1mg/ml] enthielt der Reaktionsansatz 5 mM Kaliumferricyanid (Sigma, Deisenhofen, Deutschland), 5 mM Kaliumferrocyanid (Sigma, Deisenhofen, Deutschland) und 2 mM MgCl₂ in PBS. Die mit PBS gewaschen Zellen wurden mit PBS, 2% Formaldehyd und 0,2% Glutardialdehyd 10 min bei Raumtemperatur fixiert. Anschließend wurden die Zellen mit PBS gewaschen und mit dem X-Gal-Reaktionsansatz 5 bis 24 Stunden bei 37°C inkubiert. Anhand der intrazellulären Blaufärbung konnte die β-Galactosidase nachgewiesen werden.

### 2.7 Transduktion von T-Zellen mit MLV(SIVagm3)-Vektoren

4 x 10⁵ TELCeB6/*rev*-Zellen wurden in Zellkulturschalen mit einem Durchmesser von 35 mm ausgesät und 24 Stunden später mit den SIVagm *env*-Konstrukten transfiziert. Am darauffolgenden Tag wurden die Medien der Transfektanden erneuert. Sodann wurden Kokultivierungsgefäße (Costar, Cambridge, USA) in die Kulturschalen der Verpackungszellen eingesetzt, welche den freien Austausch von Medien und den darin enthaltenden Vektoren erlauben, den direkten Zell-Zellkontakt aber unterbinden. In diese Einsätze wurden je 10⁶ T-Zellen der Linie Molt4.8 ausgesät und zusammen mit den Transfektanden zwei Tage kultiviert. Anschließend wurden die T-Zellen zwei weitere Tage expandiert und abschließend mittels X-Gal-Test auf die Expression des Reportergens *lacZ* untersucht.

Die Transfektion aller SIVagm *env*-Varianten, deren intrazelluläre Domäne nicht länger als 19 Aminosäuren ist, führten zur Bildung von pseudotypisierten MLV-Vektoren, welche die T-Zellen erfolgreich transduzierten. Die Varianten *wt env* und *Δ36env* erzeugten keine meßbaren Mengen dieser Vektoren. Als Positivkontrolle wurde das Plasmid pHIT456 (Soneoka et al., *Nucl. Acid*. *Research* 23 (1995), 628-633) transfiziert, welches das *env-*Gen des amphotropen MLV kodiert.

### Beispiel 3:

### 3.1 Etablierung stabil transfizierter Verpackungszellklone

Zur Herstellung hochtitriger Vektor-Stocks wurden TELCeB6-Zellen mit den entsprechenden *env*-Konstrukten stabil transfiziert und anschließend selektioniert. Die Selektionsmedien entsprachen den zur Kultivierung benutzten Medien, enthielten aber zusätzlich das Neomycin-Analogon G418 (800 µg/ml) zur Selektion von neo⁺-Zellen (HIV-1 *env*-Konstrukte) oder Hygromycin B (200 µg/ml) zur Selektion von hyg⁺-Zellen (SIVagm *env*-Konstrukte). Die Antibiotika wurden von Sigma (Deisenhofen, Deutschland) bezogen. Die Selektion transfizierter Zellen begann zwei Tage nach Transfektion und wurde etwa zehn Tage fortgeführt, bis sich Zellkolonien von Einzelzellklonen gebildet hatten. Die klonalen Zellen wurden mit einer Eppendorfpipette von der Kulturschale abgelöst und resuspendiert. Diese Einzelzellklone wurden zunächst in 24-Well-Platten expandiert und auf die Bildung von Vektoren mittels Titration auf geigneten Zielzellen untersucht.

### 3.2 Erzeugung von pseudotypisierten MLV-Vektoren

Die von den Verpackungszellen produzierten Vektoren wurden wie folgt präpariert: Die Medien konfluenter Verpackungszellkulturen in großen Zellkulturflaschen (800 ml) wurden abgenommen. Die Zellen wurden mit 15 ml frischen Medium beschichtet und übernacht kultiviert. Anschließend wurden die Überstände "zellfrei" durch einen 0,45 µm-Spritzenfilter filtriert und entweder direkt zur Transduktion eingesetzt oder in flüssigem Stickstoff gelagert.

### 3.3 Bestimmung der Vektortiter

Zur Bestimmung der Vektormengen in Überständen der Verpackungszellen wurden diese in verschiedenen Verdünnungen mit frischem Kulturmedium in einem Gesamtvolumen von 1 ml zur Transduktion permissiver Zielzellen (Molt4.8, C8166, Jurkat, HeLaCD4⁺) eingesetzt. Die gewählten Verdünnungen waren 1:1, 1:10, 1:100, 1:1000. Die adhärenten Zielzellen HeLa CD4⁺ wurden in einer Dichte von 2 x 10⁵ Zellen pro Zellkulturschale (35 mm Durchmesser) einen Tag vor Transduktionsbeginn ausgesät. Vor Zugabe der vektorhaltigen Medien wurden die Zellen mit PBS gewaschen und zwei Stunden bei 37°C in Gegenwart der Vektoren inkubiert. Anschließend wurden die Überstände entfernt, die Zellen erneut gewaschen und anschließend zwei weitere Tage expandiert, bevor die transduzierten Zellen mittels X-Gal-Test gefärbt wurden. Suspensionszellen wurden einen Tag vor Transduktionsbeginn mit frischen Medien versorgt. Zur Transduktion wurden je 10⁶ Zellen in üblicher Weise pelletiert (abzentrifugiert) und direkt in den vektorhaltigen Verdünnungen resuspendiert. Nach zwei Stunden wurden die Zellen gewaschen und nach zweitägiger Expansion ebenfalls mittels X-Gal-Test auf den erfolgten Gentransfer untersucht. Die so gefärbten, *LacZ*⁺-Zellen wurden unter Verwendung eines Lichtmikroskopes (Axiovert 35, Carl Zeiss, Jena) in 10 bis 20 Gesichtsfelder gezählt und auf die Gesamtfläche der Kulturschale hochgerechnet. Die Verdünnung der vektorhaltigen Überstände wurde berücksichtigt und der Titer in cfu/ml angegeben. Molt4.8-Zellen erlaubten die besten Transduktioneffizienzen, wogegen die T-Zellinien Jurkat und C8166 wesentlich schlechter zu transduzieren waren. Die HeLaCD4+-Zellen scheinen kaum permissiv für die MLV(SIVagm)-Vektoren zu sein. Die MLV(HIV-1)-Vektoren waren dagegen in der Lage, alle getesteten CD4+ Zellinien effizient zu transduzieren.

### Beispiel 4:

### Nachweis der CD4-abhängigen Transduktion mittels MLV(SIVagm)-Vektoren

Die T-Zellinie Molt4.8 wurde 30 min vor Zugabe der MLV(SIVagm3) Vektoren in Medien gehalten, die verschieden Konzentrationen (0 µg/ml, 0,625 µg/ml, 1,25 µg/ml, 2,5 µg/ml, 5 µg/ml) des CD4-bindenden monoklonalen Antikörpers IOT4a (Firma dianova, Hamburg) enthielten. Der Antikörper inhibiert den Eintritt verschiedener HIV- und SIV-Isolate durch die Blockade des zellulären Rezeptors CD4; vgl. Sattentau et al., *Science* 234 (1986), 1120-1127. Die T-Zellen wurden anschließend mittels der MLV(SIVagm)-Vektoren in Gegenwart der vorstehend genannten Konzentrationen des anti-CD4-Antikörpers IOT4a transduziert. Anschließend wurden die Zellen gewaschen und zwei Tage später mittels X-Gal-Test auf den erfolgten Gentransfer untersucht. Als Positivkontrolle wurden Zielzellen in Abwesenheit der Antikörper transduziert und als Referenz (100%) gewählt. Es zeigte sich eine eindeutig konzentrationsabhängige Korrelation der Effizienz des Gentransfers mit der eingesetzten Menge der Antikörper.
ALLGEMEINE ANGABEN :
   ANMELDER :
      NAME: Bundesrepublik Deutschland, letztvertreten durch den Präsidenten des Paul Ehrlich Instituts
      STRASSE: Paul Ehrlich Str. 51-59
      ORT: Langen
      LAND: Deutschland
      POSTLEITZAHL: 63225
   BEZEICHNUNG DER ERFINDUNG:
      Retrovirale Vektoren, Verfahren zu ihrer Herstellung und ihre
      Verwendung zur Genübertragung in CD4-positive Zellen
   ANZAHL DER SEQUENZEN: 12
   COMPUTERLESBARE FASSUNG:
      DATENTRÄGER: Diskette
      COMPUTER: IBM PC-kompatibel
      BETRIEBSSYSTEM: Windows 3.11
      SOFTWARE:PATENTIN Ausgabe #1.0, Version #1.25
ANGABEN ZU SEQ ID-NO:1:
   SEQUENZCHARAKTERISTIKA:
      LÄNGE: 25 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:1: CTAGCTAGCA TGCCCCTAGG ATCAGAAGAA AGAAG 25
ANGABEN ZU SEQ ID-NO:2:
   SEQUENZCHARAKTERISTIKA:
      LÄNGE: 23 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:2: CCGCTCGAGC TAATTAAGGA TTCCTTCAAG GCC 23
ANGABEN ZU SEQ ID-NO:1:
   SEQUENZCHARAKTERISTIKA:
      LÄNGE: 30 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:3 CAAGGCTGAG ACAAGCTTGG TGTCACTTCC 30 :
ANGABEN ZU SEQ ID-NO:4:
   SEQUENZCHARAKTERISTIKA:
      LÄNGE: 48 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:4: GTTAGGCAGG GTTACGCGGC CGCTTAACCA CAGATCCATA TCCACCCG 48
ANGABEN ZU SEQ ID-NO:5:
   SEQUENZCHARAKTERISTIKA:
      LÄNGE: 50 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:5: CGGGTGGATA TGGATCTGTG GTTAAGCGGC CGCCTAACCC TGCCTAACCC 50
ANGABEN ZU SEQ ID-NO:6:
   SEQUENZCHARAKTERISTIKA:
      LÄNGE: 48 Basenpaare
      ART: Nucleinsäure
      STRANGFORM: Einzelstrang
      TOPOLOGIE: linear
   MOLEKÜLTYP: synthetisches Oligonucleotid
   SEQUENZBESCHREIBUNG: SEQ ID-NO:6: TACTCTCCTC TTTCTCGCGG CCGCTAAATC CACCCGTGGA AGGGACAG 48
ANGABEN ZU SEQ ID-NO:7:
   SEQUENZCHARAKTERISTIKA:
      LÄNGE: 53 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:7: TCCCTTCCAC GGGTGGATTT AGCGGCCGCG AGAAAGAGGA GAGTAACCCT GCC 53
ANGABEN ZU SEQ ID-NO:8:
   SEQUENZCHARAKTERISTIKA:
      LÄNGE: 41 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:8 ATCCACCCGT GGAAGGGCGG CCGCTAAAAC GCAGAAGGGC C 41
ANGABEN ZU SEQ ID-NO:9:
   SEQUENZCHARAKTERISTIKA:
      LÄNGE: 43 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:9: CCCTTCTGCG TTTTAGCGGC CGCCCTTCCA CGGGTGGATA TGG 43
ANGABEN ZU SEQ ID-NO:10:
   SEQUENZCHARAKTERISTIKA:
      LÄNGE: 56 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:10: AAAAGGAAAA GCGGCCGCTC GATTAGTCCA ATTTGTTAAA GACAGGATAT CAGTGG 56
ANGABEN ZU SEQ ID-NO:11:
   SEQUENZCHARAKTERISTIKA:
      LÄNGE: 51 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:11: AAAAGGAAAA GCGGCCGCGA CAGGATATCA GTGGTCCAGG CTCTAGTTTT G 51
ANGABEN ZU SEQ ID-NO:12:
   SEQUENZCHARAKTERISTIKA:
      LÄNGE: 49 Basenpaare
      ART: Nucleinsäure
      STRANGFORM: Einzelstrang
      TOPOLOGIE: linear
   MOLEKÜLTYP: synthetisches Oligonucleotids
   SEQUENZBESCHREIBUNG: SEQ ID-NO:12:

AAAAGGAAAA GCGGCCGCCT ATGGCTCGTA CTCTATAGGC TTCAGCTGG 49

## Patentansprüche

1. Retrovirale Vektoren umfassend Viruskerne und aus Oberflächenhüllproteinen und transmembranen Hüllproteinen bestehende Virushüllen, wobei die Viruskerne vom murinen Leukämievirus (MLV) und. die Virushüllen von menschlichen Immunschwächeviren (HIV) oder Affen-Immunschwächeviren (SIV) stammen, und die Virusküllen das Vollängen- Oberflächemprotein und eine Verkürzte Forms des Hüllproteins enthallen.

2. Retrovirale Vektoren nach Anspruch 1, wobei die Virushüllen vom menschlichen Immunschwächevirus 1 oder 2 (HIV-1 bzw. HIV-2) oder Affen-Immunschwächevirus *Cerecopithecus aethiops* (SIVagm) oder *Macaca mulatta* (SIVmac) oder *Pan troglodydytes* (SIVcpz) oder *Cerecopithecus mitis* (SIVsyk) oder *Papio sphinx* (SIVmnd) oder *Cercocebus atys* (SIVsm) oder *Macaca nemestrina* (SIVmne) stammen.

3. Retrovirale Vektoren nach Anspruch 1 oder 2, wobei die Virushüllen das Vollängen-Oberflächenprotein und eine verkürzte Form des transmembranen Hüllproteins enthalten, das durch den C-Terminus oder ein beliebiges Fragment des transmembranen Proteins des murinen Leukämievirus (MLV) oder eines anderen Retrovirus verlängert worden ist.

4. Verfahren zur Herstellung von Verpackungszellen, die retrovirale Vektoren nach einem der Ansprüche 1 bis 3 bilden, umfassend das Transfizieren einer Verpackungszelle (*gag*-, *pol-* und Expressionskonstrukt-positiv), die vom MLV stammende Hüllproteine produziert oder nicht produziert (*env*-negativ), mit einem Expressionsgen, das die genetische Information für Hüllproteine von HIV oder SIV enthält (*env*-positiv).

5. Verfahren zur Herstellung von Verpackungszellen, die retrovirale Vektoren nach einem der Ansprüche 1 bis 3 bilden, umfassend das Transfizieren einer Zelle mit Expressionsgenen für *gag* und *pol*, und einem Expressionskonstrukt, umfassend ein Verpackungssignal (psi) und die zu überführende genetische Information, und einem Expressionsgen, das die genetische Information für Hüllproteine von HIV oder SIV enthält.

6. Verfahren nach Anspruch 4, wobei als MLV-*env*-negative Verpackungszelle die Zellinie TELCeB6 verwendet wird.

7. Verfahren nach einem der Ansprüche 4 bis 6, wobei als *env*-Expressionsgen pLβAc/*env*-Tr712-neo oder pRep Δ16 *env,* pRep Δ7 *env,* pRep *Δ*0 *env,* pRep Δ7MLV *env* oder pRep Δ0MLV *env* (Abb. 9 und 10) verwendet wird.

8. Verpackungszellen, erhältlich nach dem Verfahren nach einem der Ansprüche 4 bis 7.

9. Verwendung der retroviralen Vektoren nach einem der Ansprüche 1 bis 3 zur Herstellung eines Arzneimittels

10. Verwendung der retroviralen Vektoren nach einem der Ansprüche 1 bis 3 zur Herstellung eines Arzneimittels zur Überführung von Genen in CD4-positive Zellen von Säugern.

11. Verwendung der retroviralen Vektoren nach einem der Ansprüche 1 bis 3 zur Herstellung eines Arzneimittesl zur Überführung von Genen in CD4-positive Zellen des Menschen.

12. Verwendung der retroviralen Vektoren nach einem der. Ansprüche 1 bis 3 zur Herstellung eines Arzneimittels zur gentechnischen Modifizierung von CD4-positiven Zellen oder Zur Herstellung eines Arzneimittels für die Gentherapie

## Claims

1. Retroviral vectors comprising viral cores and viral envelopes consisting of surface envelope proteins and transmembrane envelope proteins, wherein the viral cores are derived from murine leukemia virus (MLV) and the viral envelopes are derived from human immunodeficiency viruses (HIV) or simian immunodeficiency viruses (SIV) and wherein the viral envelopes contain the full length surface protein and a truncated form of the envelope protein.

2. Retroviral vectors according to claim 1, wherein the viral envelopes are derived from human immunodeficiency virus 1 or 2 (HIV-1 and HIV-2, respectively) or simian immunodeficiency virus *Cerecopithecus aethiops* (SIVagm) or *Macaca mulatta* (SIVmac) or *Pan troglodytes* (SIVcpz) or *Cerecopithecus mitis* (SIVsyk) or *Papio sphinx* (SIVmnd) or *Cercocebus atys* (SIVsm) or *Macaca nemestrina* (SIVmne).

3. Retroviral vectors according to claim 1 or 2, wherein the viral envelopes contain the full length surface protein and a truncated form of the transmembrane envelope protein, which has been extended by the C-terminus or any fragment of the transmembrane protein from murine leukemia virus (MLV) or another retrovirus.

4. Method for preparing packaging cells generating retroviral vectors according to any one claims 1 to 3, comprising transfecting a packaging cell (*gag*, *pol* and expression construct positive) which produces or does not produce *(env* negative) envelope proteins derived from MLV with an expression gene which contains the genetic information for envelope proteins from HIV or SIV *(env* positive).

5. Method for preparing packaging cells generating retroviral vectors according to any one of claims 1 to 3 comprising transfecting a cell with expression genes for *gag* and *pol* and an expression construct comprising a packaging signal (psi) and the genetic information to be transferred and an expression gene which contains the genetic information for envelope proteins from HIV or SIV.

6. Method according to claim 4, wherein the cell line TELCeB6 is used as MLV *env* negative packaging cell.

7. Method according to any one of claims 4 to 6, wherein pLβAc/env-Tr712-neo or pRep Δ16 *env,* pRep Δ7 *env*, pRep Δ0 *env,* pRep Δ7MLV *env* or pRep ΔOMLV *env* (Fig. 9 and 10) is used as *env* expression gene.

8. Packaging cells obtainable by the method according to any one of claims 4 to 7.

9. Use of the retroviral vectors according to any one of claims 1 to 3 for the preparation of a medicament.

10. Use of the retroviral vectors according to any one of claims 1 to 3 for the preparation of a medicament for transferring genes into CD4 positive mammalian cells.

11. Use of the retroviral vectors according to any one of claims 1 to 3 for the preparation of a medicament for transferring genes into CD4 positive human cells.

12. Use of the retroviral vectors according to any one of claims 1 to 3 for the preparation of a medicament for genetically modifying CD4 positive cells or for the preparation of a medicament for gene therapy.

## Revendications

1. Vecteurs rétroviraux comprenant des noyaux de virus et des enveloppes virales consistant en des protéines d'enveloppe de surface et des protéines d'enveloppe transmembranaires, tandis que les noyaux de virus proviennent de virus de leucémie murine (MLV) et les enveloppes virales proviennent de virus de l'immunodéficience humaine (VIH) ou de virus de l'immunodéficience simien (VIS), et les enveloppes virales contiennent la protéine de surface de longueur totale et une forme raccourcie de la protéine d'enveloppe.

2. Vecteurs rétroviraux selon la revendication 1, dans lesquels les enveloppes virales proviennent de virus de l'immunodéficience humaine 1 ou 2 (VIH-1 ou VTH-2) ou de virus de l'immunodéficience simien de *Cerecopithecus aethiops* (VISagm) ou *Macaca mulatta* (VIS mac) ou *Pan troglodytes* (VIScpz) ou *Cerecopithecus mitis* (VISsyk) ou *Papio sphinx* (VISmnd) ou *Cercocebus atys* (VISsm) ou *Macaca nemestrina* (VIS mne).

3. Vecteurs rétroviraux selon la revendication 1 ou 2, dans lesquels les enveloppes virales contiennent la protéine de surface de longueur totale et une forme raccourcie de la protéine d'enveloppe transmembranaire, qui a été prolongée par le fragment de l'extrémité C terminale ou un fragment quelconque de la protéine transmembranaire du virus de la leucémie murine (MLV) ou d'un autre rétrovirus.

4. Procédé pour la préparation de cellules d'encapsulation, qui forment des vecteurs rétroviraux selon l'une des revendications 1 à 3, comprenant la transfection d'une cellule d'encapsulation (positive pour *gag*, *pol* et un construit d'expression), qui produit ou ne produit pas (négative pour *env*) des protéines d'enveloppe provenant de MLV, avec un gène d'expression qui contient (positif pour *env*) l'information génétique pour des protéines d'enveloppe de VIH ou VIS.

5. Procédé pour la préparation de cellules d'encapsulation, qui forment des vecteurs rétroviraux selon l'une des revendications 1 à 3, comprenant la transfection d'une cellule avec des gènes d'expression pour *gag* et*pol,* et un construit d'expression, comportant un signal d'encapsulation (psi) et l'information génétique à transférer, et un gène d'expression qui contient l'information génétique pour des protéines d'enveloppe de VIH ou VIS.

6. Procédé selon la revendication 4, dans lequel on utilise comme cellule d'encapsulation négative pour *env* de MLV la lignée cellulaire TELCeB6.

7. Procédé selon l'une des revendications 4 à 6, dans lequel on utilise comme gène d'expression d'*env* pLβAc/*env*-Tr712-néo ou pRep *Δ*16 *env,* pRep Δ7 *env,* pRep Δ0 *env,* pRep Δ7MLV *env* ou pRep ΔOMLV *env* (Figs. 9 et 10).

8. Cellules d'encapsulation, pouvant être obtenues par le procédé selon l'une des revendications 4 à 7.

9. Utilisation des vecteurs rétroviraux selon l'une des revendications 1 à 3 pour la préparation d'un médicament.

10. Utilisation des vecteurs rétroviraux selon l'une des revendications 1 à 3 pour la préparation d'un médicament pour le transfert de gènes dans des cellules CD4-positives de mammifères.

11. Utilisation des vecteurs rétroviraux selon l'une des revendications 1 à 3 pour la préparation d'un médicament pour le transfert de gènes dans des cellules CD4-positives humaines.

12. Utilisation des vecteurs rétroviraux selon l'une des revendications 1 à 3 pour la préparation d'un médicament pour la modification par génie génétique de cellules CD4-positives ou pour la préparation d'un médicament pour la thérapie génique.
